# EUROPEAN PATENT APPLICATION

(11) **EP 3 196 291 A1**
(43) Date of publication of application: **26.07.2017**
(21) Application number: 15835463.9
(22) Date of filing: 30.06.2015
(51) Int. Cl.: C12M 1/34, C12Q 1/68, G01N 33/50, G06F 19/18, G06F 19/00

(54) **INSPECTION NOTICE OUTPUT DEVICE, INSPECTION NOTICE OUTPUT METHOD, INSPECTION NOTICE OUTPUT PROGRAM AND GENE CHROMOSOME INSPECTION SYSTEM**

(30) Priority: 25.08.2014 JP 2014170238
(71) Applicant: Fujifilm Corporation, Minato-ku Tokyo 106-8620 (JP)
(72) Inventor: TSUJIMOTO, Takayuki, Kanagawa 258-8538 (JP)
(74) Representative: Klunker IP Patentanwälte PartG mbB
(86) International application number: PCT/JP2015/068857
(87) International publication number: WO 2016/031385

(57) **Abstract**

There are provided an examination notification output device, an examination notification output method, an examination notification output program, and a gene chromosome examination system capable of outputting information, which is an indicator for determining whether or not a re-examination is required, in a case where it is determined that a re-examination is required for a gene chromosome examination. An examination notification output device that outputs an examination notification of a gene chromosome examination for examining the abnormalities of chromosomes included in embryonic cells includes: an information acquisition unit that acquires information including an examination result of a gene chromosome examination; an examination notification generation unit that generates an examination notification by adding re-examination necessity determination information that is used in determining whether or not a re-examination is required; and an examination notification output unit that outputs the generated examination notification.

## Description

### BACKGROUND OF THE INVENTION

### 1. Field of the Invention

The present invention relates to an examination notification output device, an examination notification output method, an examination notification output program, and a gene chromosome examination system, and in particular to a technique of outputting a biological information analysis result, which is performed by isolating cells obtained from a body.

### 2. Description of the Related Art

Due to an improvement in the performance of a DNA sequencer that has progressed in recent years, it has become possible to perform gene analysis using a high-accuracy and convenient DNA sequencer. DNA is an abbreviation of deoxyribonucleic acid.

JP2010-525832A discloses a non-invasive prenatal diagnostic method. JP2010-525832A discloses a gene chromosome examination of acquiring a cell population including at least one type of fetal nucleated cells from maternal blood, concentrating the acquired cell population, identifying the fetal nucleated cells by labeling using specific antibodies against the fetal nucleated cells, isolating the labeled fetal nucleated cells, extracting DNA from the isolated fetal nucleated cells, amplifying the DNA, and analyzing the presence or absence of chromosome aneuploidy.

The term "fetal nucleated cells" and the term "chromosome aneuploidy" disclosed in JP2010-525832A correspond to a term "embryonic cells" and a term "numerical aberration of a specific chromosome" in this specification, respectively.

### SUMMARY OF THE INVENTION

Fetal-derived cells in maternal blood are rare. For this reason, target embryonic cells may not be able to be obtained, or a sufficient gene analysis result to determine the presence or absence of numerical aberration of a specific chromosome may not be able to be obtained even if the target embryonic cells can be obtained. Accordingly, it may be necessary to perform gene analysis or blood collection again.

Performing gene analysis again is burdensome in terms of cost and period. Collecting blood again increases a burden on the mother. In addition, even if gene analysis is performed again, a sufficient result to determine the presence or absence of numerical aberration of a specific chromosome is not necessarily obtained.

In the method disclosed in JP2010-525832A, the case where target embryonic cells cannot be obtained or the case where a sufficient gene analysis result to determine the presence or absence of numerical aberration of a specific chromosome cannot be obtained has not taken into consideration.

The present invention has been made in view of the aforementioned situation, and it is an object of the present invention to provide an examination notification output device, an examination notification output method, an examination notification output program, and a gene chromosome examination system capable of outputting information, which is an indicator for determining whether or not a re-examination is required, in a case where it is determined that a re-examination is required for a gene chromosome examination.

In order to achieve the aforementioned object, according to a first aspect, there is provided an examination notification output device that outputs an examination notification of a gene chromosome examination for examining abnormalities of chromosomes included in embryonic cells. The examination notification output device comprises: an information acquisition unit that acquires information including an examination result of a gene chromosome examination; an examination notification generation unit that generates an examination notification by adding re-examination necessity determination information, which is used in determining whether or not a re-examination is required, to the examination result; and an examination notification output unit that outputs the generated examination notification.

According to the first aspect, an examination notification is generated by adding the re-examination necessity determination information, which is used in determining whether or not a re-examination is required, to the examination result, and the examination notification is output. Therefore, in the determination regarding whether or not to perform re-examination, it is possible to use the re-examination necessity determination information as an indicator.

According to a second aspect, in the examination notification output device according to the first aspect, the information acquisition unit acquires information including at least one of a number of used cells indicating a number of cells used in the gene chromosome examination, a gestational age of a mother at the time of the gene chromosome examination, a number of candidate cells indicating a number of cells as candidates that are used in the gene chromosome examination, an amount of blood collection, an amount of DNA, an amount of chromosomes, or a gene dosage, and the examination notification generation unit adds at least one of the pieces of information acquired, as the re-examination necessity determination information, to the examination notification.

According to the second aspect, the information of at least one of the number of used cells, the gestational age of the mother at the time of gene chromosome examination, the number of candidate cells, the amount of blood collection, the amount of DNA, the amount of chromosomes, or the gene dosage can be used as an indicator for determining whether or not a re-examination is required.

According to a third aspect, in the examination notification output device according to the first or second aspect, the examination notification generation unit adds re-examination success possibility information indicating a possibility that re-examination will be successful, as the re-examination necessity determination information, to the examination notification.

According to the third aspect, since the examination notification including the re-examination success possibility information as re-examination necessity determination information is output, whether or not the possibility of success of re-examination is high can be grasped at a glance from the re-examination success possibility information.

According to a fourth aspect, in the examination notification output device according to the third aspect, the information acquisition unit acquires reason information indicating a determination reason for the re-examination success possibility information, and the examination notification generation unit adds the acquired reason information to the examination notification.

According to the fourth aspect, it is possible to use the reason information indicating the reason for the re-examination success possibility information as an indicator for determining whether or not to perform a re-examination. In addition, in a case where a re-examination is performed, it is possible to set the conditions of the re-examination based on the reason information.

In the fourth aspect, it is preferable to acquire information of at least one of the number of used cells and the gestational age of the mother at the time of gene chromosome examination and add the acquired information to the examination notification.

According to a fifth aspect, in the examination notification output device according to any one of the first to fourth aspects, the information acquisition unit acquires information of a gestational age of the mother at the time of examination notification output, and the examination notification generation unit adds the acquired gestational age of the mother at the time of examination notification output to the examination notification.

According to the fifth aspect, since the gestational age at the time of examination notification output is added to the examination notification, the gestational age at the time of examination notification output can be used as an indicator for determining whether or not a re-examination is required. In addition, in a case where a re-examination is performed, it is possible to set the conditions of the re-examination based on the information of the gestational age at the time of examination notification output.

In the fifth aspect, the time of examination notification output may be a point in time when an examination result is displayed for a person to be examined. In a case where it is not possible to calculate a point in time when an examination result is displayed for a person to be examined, the time of examination notification output may be a point in time when the examination notification is acquired when displaying the examination notification for the person to be examined, a point in time when the examination notification is generated by the examination notification generation unit, or a point in time when the examination result is output by the examination result output unit.

According to a sixth aspect, in the examination notification output device according to any one of the first to fifth aspects, the information acquisition unit acquires information of a period required for a re-examination in a case where the re-examination is performed, and the examination notification generation unit adds the acquired period, which is required for the re-examination in a case where the re-examination is performed, to the examination notification.

According to the sixth aspect, since a period required for a re-examination in a case where the re-examination is performed is added to the examination notification, the information of a period required for a re-examination can be used as an indicator when determining the conditions of the re-examination, such as performing the re-examination early.

According to a seventh aspect, in the examination notification output device according to any one of the first to sixth aspects, the information acquisition unit acquires information that is an indicator for setting conditions of a re-examination in a case where the re-examination is performed, and the examination notification generation unit adds the acquired information, which is an indicator for setting the conditions of the re-examination, to the examination notification.

According to the seventh aspect, in a case where a re-examination is performed, it is possible to set the conditions of the re-examination, such as performing a re-examination preferentially or changing the conditions in the previous examination when performing a re-examination.

According to an eighth aspect, in the examination notification output device according to any one of the first to seventh aspects, the information acquisition unit acquires information regarding a charge in a case where a re-examination is performed, and the examination notification generation unit adds the acquired information regarding a charge to the examination notification.

According to the eighth aspect, in a case where a re-examination is performed, a response, such as making no additional charge at the time of re-examination, is possible.

According to a ninth aspect, in the examination notification output device according to any one of the first to eighth aspects, the information acquisition unit acquires information of a step of the gene chromosome examination, in which it has been determined that an examination result indicating that a re-examination is required has been obtained, and the examination notification generation unit adds the acquired information of the step of the gene chromosome examination, in which it has been determined that an examination result indicating that a re-examination is required has been obtained, to the examination notification.

According to the ninth aspect, since it is possible to determine whether or not to perform a re-examination more early, it is possible to perform a re-examination more early in a case where the re-examination is performed.

According to a tenth aspect, in the examination notification output device according to any one of the first to ninth aspects, the information acquisition unit acquires information of a step of the gene chromosome examination, from which a re-examination is started in a case where the re-examination is performed, and the examination notification generation unit adds the acquired information of the step of the gene chromosome examination, from which a re-examination is started, to the examination notification.

According to the tenth aspect, in a case where a re-examination is performed, it is possible to start the re-examination from the intermediate step in the gene chromosome examination.

According to an eleventh aspect, in the examination notification output device according to any one of the first to tenth aspects, in a case where it is determined that a re-examination is required based on the acquired examination result, an examination notification including the re-examination necessity determination information is generated.

According to the eleventh aspect, an examination notification is generated by adding the re-examination necessity determination information to the examination result in a case where a re-examination is required, and the examination notification is output. Therefore, in the determination regarding whether or not to perform re-examination in a case where a re-examination is required, it is possible to use the re-examination necessity determination information as an indicator.

According to a twelfth aspect, in the examination notification output device according to the eleventh aspect, in a case where the acquired examination result corresponds to neither a normal range nor an abnormal range or in a case where the acquired examination result does not correspond to an examination result in embryonic cells, the examination notification generation unit generates an examination notification including re-examination necessity determination information, which is used in determining whether or not a re-examination is required, in a case where it is determined that a re-examination is required based on the acquired examination result.

According to the twelfth aspect, the case where the acquired examination result corresponds to neither the normal range nor the abnormal range or the case where the acquired examination result does not correspond to the examination result in embryonic cells can be treated as a case where a re-examination is required.

According to a thirteenth aspect, in the examination notification output device according to the eleventh or twelfth aspect, the information acquisition unit acquires information indicating a case where it has been determined that a re-examination is required (information indicating that it has been determined that a re-examination is required).

According to the thirteenth aspect, in a case where the information indicating a case where it has been determined that a re-examination is required is acquired, an examination notification can be generated by adding the re-examination necessity determination information to the examination result.

According to a fourteenth aspect, the examination notification output device according to any one of the eleventh to thirteenth aspects further comprises a determination unit that determines whether or not a re-examination is required based on the acquired information.

According to the fourteenth aspect, whether or not a re-examination is required can be automatically determined based on the acquired information.

According to a fifteenth aspect, there is provided an examination notification output method for outputting an examination notification of a gene chromosome examination for examining abnormalities of chromosomes included in embryonic cells. The examination notification output method includes: an information acquisition step of acquiring information including an examination result of a gene chromosome examination; an examination notification generation step of generating an examination notification by adding re-examination necessity determination information, which is used in determining whether or not a re-examination is required, to the examination result; and an examination notification output step of outputting the generated examination notification.

In the fifteenth aspect, preferably, in the information acquisition step, information including at least one of the number of used cells indicating the number of cells used in the gene chromosome examination, a gestational age of a mother at the time of the gene chromosome examination, the number of candidate cells indicating the number of cells as candidates that are used in the gene chromosome examination, an amount of blood collection, an amount of DNA, an amount of chromosomes, or a gene dosage is acquired. Preferably, in the examination notification generation step, the acquired information is added to the examination notification as the re-examination necessity determination information.

In the fifteenth aspect, preferably, in the examination notification generation step, re-examination success possibility information indicating a possibility that re-examination will be successful is added to the examination notification as the re-examination necessity determination information.

According to the aspect, preferably, in the information acquisition step, reason information indicating a determination reason for the re-examination success possibility information is acquired. Preferably, in the examination notification generation step, the acquired reason information is added to the examination notification.

In the fifteenth aspect, preferably, in the information acquisition step, information of a gestational age of the mother at the time of examination notification output is acquired. Preferably, in the examination notification generation step, the acquired gestational age of the mother at the time of examination notification output is added to the examination notification.

In the fifteenth aspect, preferably, in the information acquisition step, information of a period required for a re-examination in a case where the re-examination is performed is acquired. Preferably, in the examination notification generation step, the acquired period required for the re-examination in a case where the re-examination is performed is added to the examination notification.

In the fifteenth aspect, preferably, in the information acquisition step, information that is an indicator for setting conditions of a re-examination in a case where the re-examination is performed is acquired. Preferably, in the examination notification generation step, the acquired information, which is an indicator for setting the conditions of the re-examination, is added to the examination notification.

In the fifteenth aspect, preferably, in the information acquisition step, information regarding a charge in a case where a re-examination is performed is acquired. Preferably, in the examination notification generation step, the acquired information regarding a charge is added to the examination notification.

In the fifteenth aspect, preferably, in the information acquisition unit, information of a step of the gene chromosome examination, in which it has been determined that an examination result indicating that a re-examination is required has been obtained, is acquired. Preferably, the examination notification generation unit adds the acquired information of the step of the gene chromosome examination, in which it has been determined that an examination result indicating that a re-examination is required has been obtained, to the examination notification.

In the fifteenth aspect, preferably, in the information acquisition step, information of a step of the gene chromosome examination, from which a re-examination is started in a case where the re-examination is performed, is acquired. Preferably, in the examination notification generation step, the acquired information of the step of the gene chromosome examination from which a re-examination is started is added to the examination notification.

In the fifteenth aspect, preferably, in a case where it is determined that a re-examination is required based on the acquired examination result, an examination notification including the re-examination necessity determination information is generated.

In the fifteenth aspect, preferably, in the examination notification generation step, in a case where the acquired examination result corresponds to neither a normal range nor an abnormal range or in a case where the acquired examination result does not correspond to an examination result in embryonic cells, an examination notification including re-examination necessity determination information, which is used in determining whether or not a re-examination is required, is generated in a case where it is determined that a re-examination is required based on the acquired examination result.

In the fifteenth aspect, preferably, in the information acquisition step, information indicating a case where it has been determined that a re-examination is required is acquired.

In the fifteenth aspect, it is preferable to further include a determination step of determining whether or not a re-examination is required based on the acquired information.

According to a sixteenth aspect, there is provided an examination notification output program for outputting an examination notification of a gene chromosome examination for examining abnormalities of chromosomes included in embryonic cells. The program causes a computer to function as: information acquisition means for acquiring information including an examination result of a gene chromosome examination; examination notification generation means for generating an examination notification by adding re-examination necessity determination information, which is used in determining whether or not a re-examination is required, to the examination result; and examination notification output means for outputting the generated examination notification.

In the sixteenth aspect, it is preferable to cause a computer to function as means corresponding to each unit according to any one of the second to thirteenth aspects.

According to a seventeenth aspect, there is provided a gene chromosome examination system for examining abnormalities of chromosomes included in embryonic cells. The gene chromosome examination system comprises: an examination unit that performs a gene chromosome examination of acquiring a blood sample collected from a mother, recovering candidate cells, which are candidates for embryonic cells, from the acquired blood sample, and performing gene analysis using the recovered candidate cells; and an examination notification output device that outputs an examination notification of the gene chromosome examination performed by the examination unit. The examination notification output device comprises: an information acquisition unit that acquires information including an examination result of a gene chromosome examination; an examination notification generation unit that generates an examination notification by adding re-examination necessity determination information, which is used in determining whether or not a re-examination is required, to the examination result; and an examination notification output unit that outputs the generated examination notification.

According to an eighteenth aspect, in the gene chromosome examination system according to the seventeenth aspect, the examination notification output device includes the examination notification output device according to any one of the second to fourteenth aspects.

According to the present invention, since the examination notification is generated by adding the re-examination necessity determination information, which is used in determining whether or not a re-examination is required, to the examination result, and the examination notification is output, it is possible to use the re-examination necessity determination information as an indicator in the determination regarding whether or not to perform re-examination.

### BRIEF DESCRIPTION OF THE DRAWINGS

Fig. 1 is a flowchart showing the examination procedure of a gene chromosome examination.
Fig. 2 is a flowchart showing the work flow of a cell isolation step.
Fig. 3 is a flowchart showing the work flow of a gene analysis step.
Fig. 4 is an overall configuration diagram showing the schematic configuration of a gene chromosome examination system to which an examination notification output device according to an embodiment of the present invention is applied.
Fig. 5 is a block diagram showing the configuration of an information processing system in a laboratory shown in Fig. 4.
Fig. 6 is a block diagram of a gene chromosome examination system to which a client server type computer system is applied.
Fig. 7 is a block diagram showing the configuration of an examination notification output device shown in Fig. 5.
Fig. 8 is an explanatory diagram showing an example of the examination notification sent in a case where an examination result corresponds to neither a normal range nor an abnormal range.
Fig. 9 is an explanatory diagram showing another example of the examination notification sent in a case where it is determined that a re-examination is required.
Fig. 10 is an explanatory diagram showing another example of the examination notification in a case where it is determined that a re-examination is required.
Fig. 11 is an explanatory diagram showing an example of the notification of an examination result in a case where a re-examination is not required in a case where the fetus is a female.
Fig. 12 is an explanatory diagram showing an example of the notification of an examination result in a case where a re-examination is not required in a case where the fetus is a male.
Fig. 13 is a flowchart showing the control procedure of an examination notification output method.
Fig. 14 is an explanatory diagram of an examination notification including reason information indicating the determination reason for re-examination success determination.
Fig. 15 is an explanatory diagram of an examination notification including supplementary information that supplements re-examination necessity determination information.
Fig. 16 is an explanatory diagram of another example of supplementary information.
Fig. 17 is an explanatory diagram of another example of supplementary information.
Fig. 18 is an explanatory diagram of another example of supplementary information.
Fig. 19 is an explanatory diagram of another example of supplementary information.

### DESCRIPTION OF THE PREFERRED EMBODIMENTS

Hereinafter, preferred embodiments of the present invention will be described in detail with reference to the accompanying diagrams.

### [Explanation of examination procedure of gene chromosome examination]

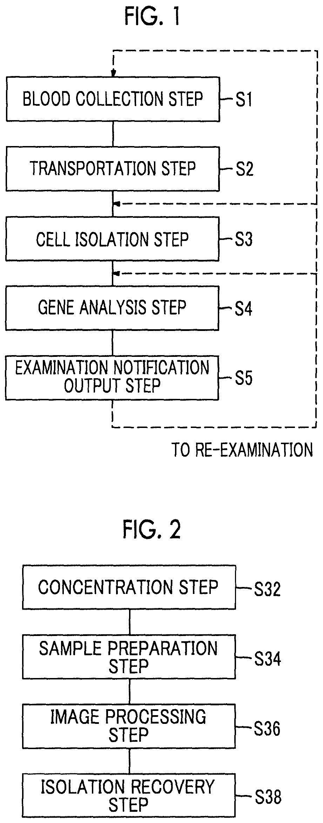

Fig. 1 is a flowchart showing the examination procedure of a gene chromosome examination. The gene chromosome examination, which will be described below, is intended to examine the abnormalities of chromosomes contained in embryonic cells. In the following explanation, embryonic nucleated red blood cells are exemplified as embryonic cells. As a cell, a part of a cell that can be a target to be examined, for example, a cell debris, may be applied.

The gene chromosome examination whose examination procedure is shown in Fig. 1 includes a blood collection step S1, a transportation step S2, a cell isolation step S3, a gene analysis step S4, and an examination notification output step S5.

The blood collection step S1 is a step of collecting blood from the mother, and can be understood as a sample collection step of collecting a blood sample. In the blood collection step S1, for example, a nurse or the like in the obstetrics and gynecology department of the hospital collects blood directly from the mother.

The transportation step S2 is a step of transporting a blood sample collected in the hospital to the laboratory. The term "laboratory" is used as a general term of a location, a facility, an institution, a company, and the like where sample examination is performed. As examples of the laboratory, an examination agency, an examination center, an examination company, and a medical institute specializing in the business of sample examination in response to outsourcing from medical institutions, such as hospitals, are assumed. In addition, the laboratory may be provided within the hospital.

The cell isolation step S3 is a step of isolating nucleated red blood cells from a blood sample to recover the nucleated red blood cells as candidate cells. In the nucleated red blood cells recovered in the cell isolation step S3, embryonic nucleated red blood cells and maternally derived nucleated red blood cells are mixed, and the embryonic nucleated red blood cells and the maternally derived nucleated red blood cells are not distinguished.

Based on the examination result obtained in the gene analysis step S4 to be described later, it is determined whether or not embryonic nucleated red blood cells have been recovered, and the presence or absence of numerical aberration of a specific chromosome is determined in a case where embryonic nucleated red blood cells have been recovered.

The gene analysis step S4 is an analysis step of analyzing the numerical aberration of a specific chromosome from the nucleated red blood cells recovered by isolation. In the gene analysis step S4, it is determined whether or not embryonic nucleated red blood cells have been recovered. In addition, in the gene analysis step S4, analysis to detect the numerical aberration of a specific chromosome is performed. As an example of a specific chromosome, a chromosome 18 or a chromosome 21 can be mentioned. As the numerical aberration of a specific chromosome, the presence or absence of at least one of monosomy, trisomy, or tetrasomy is checked.

Trisomy is a karyotype with three chromosomes (normally, two chromosomes make a pair). As targets of prenatal genetic examination, in the present circumstances, three chromosomes of a chromosome 13, a chromosome 18, and a chromosome 21 are assumed. Monosomy is a karyotype with one chromosome (normally, two chromosomes make a pair). Tetrasomy is a karyotype with four chromosomes (normally, two chromosomes make a pair).

The examination notification output step S5 is a step of generating an examination notification based on the examination result of the gene chromosome examination and outputting the generated examination notification. The examination notification is output in the form of electronic data or in a printed form. The examination notification output in the form of electronic data is visualized by being displayed on a display device or by being printed by a printing device.

Here, information indicating whether embryonic nucleated red blood cells have been recovered and information indicating the presence or absence of numerical aberration of a specific chromosome are included in the examination result of a gene chromosome examination. In addition, the amount of DNA is applied as information indicating the presence or absence of the numerical aberration of a specific chromosome.

In the present embodiment, a quantitative value of DNA obtained by quantifying the amount of DNA is applied as the amount of DNA. Instead of the amount of DNA, the amount of chromosomes obtained by calculating the number of chromosomes as an evaluated value and the amount of genes obtained by calculating a gene dosage as an evaluated value can be applied. As an example of the amount of chromosomes or the amount of genes, a quantitative value obtained by quantification can be mentioned.

In a case where the examination result of embryonic nucleated red blood cells is not included in the examination result shown on the examination notification, that is, in a case where a target examination result cannot be obtained, such as a case where embryonic nucleated red blood cells that are target cells cannot be recovered or a case where it is difficult to determine the numerical aberration of a gene chromosome of the fetus, a re-examination is performed as required.

There may be a case where a re-examination is performed from the blood collection step S1 in Fig. 1, a case where a re-examination is performed from the cell isolation step S3, or a case where a re-examination is performed from the gene analysis step S4 of Fig. 1. Fig. 1 shows a transition to step in which a re-examination is started using a dotted arrow line.

Fig. 2 is a flowchart showing the work flow of the cell isolation step. The cell isolation step shown in Fig. 2 is the cell isolation step S3 shown in Fig. 1. The cell isolation step shown in Fig. 2 includes a concentration step S32, a sample preparation step S34, an image processing step S36, and an isolation recovery step S38.

The concentration step S32 is a step of concentrating nucleated red blood cells in a blood sample. As a concentration method, there is a density gradient centrifugation method, a removal method based on a magnetic separation method of leucocytes, or the like.

The sample preparation step S34 is a step of preparing a smear of the concentrated blood sample. In the sample preparation step S34, a blood sample having passed through the concentration step S32 is spread on the slide glass, dried, fixed, and stained, so that a smear as a microscopic sample is prepared.

That is, if the sample preparation step S34 is divided more finely, a collection step, a smear step, a first drying step, a staining step, and a second drying step are included in the sample preparation step S34.

The collection step is a step of collecting a blood sample having passed through the concentration step. The smear step is a step of spreading the collected blood sample on the slide glass thinly and uniformly. The first drying step is a step of drying the blood sample smeared on the slide glass before staining. The staining step is a step of staining the blood sample dried in the first drying step. As a staining method, it is possible to use Giemsa staining. The second drying step is a step of drying the blood sample after staining.

After the concentration step, a smear is obtained through the collection step, the smear step, the first drying step, the staining step, and the second drying step. The term "smear" may be referred to as a slide sample or may be simply referred to as a slide.

The image processing step S36 is a step of acquiring a scan image of the smear using a slide scanner, analyzing the obtained scan image, and detecting nucleated red blood cells. In order to detect nucleated red blood cells, a method of detecting the shape of nucleated red blood cells from an image or the like is used.

The isolation recovery step S38 is a step of detecting the position of the nucleated red blood cells on the slide glass of the smear based on the image analysis result of the scan image and isolating and recovering the nucleated red blood cells whose position has been detected.

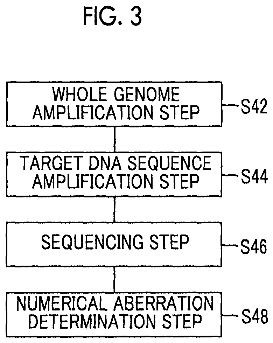

Fig. 3 is a flowchart showing the work flow of the gene analysis step. The gene analysis step shown in Fig. 3 is the gene analysis step S4 shown in Fig. 1. As shown in Fig. 3, the gene analysis step includes a whole genome amplification step S42, a target DNA sequence amplification step S44, a sequencing step S46, and a numerical aberration determination step S48.

The whole genome amplification step S42 is a step of performing a process of amplifying a whole genome from a single cell. Since one cell to several cells are not sufficient for analysis, DNA information is copied in the order of 10,000 times to a few million times.

In the target DNA sequence amplification step S44, processing for amplifying only the specific chromosome information from DNA, which needs to be amplified, using polymerase chain reaction is performed. The polymerase chain reaction may be referred to as PCR. The PCR is an abbreviation of polymerase chain reaction.

The sequencing step S46 is a step of identifying the nucleotide sequence of the mother and the nucleotide sequence of the fetus by specifying the attribution of a chromosome using a next-generation sequencer. That is, in the sequencing step S46, quantification of DNA amplified in the target DNA sequence amplification step S44 and determination of attribution are performed.

Based on the information obtained in the sequencing step S46, it is possible to determine whether or not embryonic nucleated red blood cells that are target cells have been recovered. That is, it is determined that embryonic nucleated red blood cells have been recovered in a case where two types of nucleated red blood cells derived differently have been recovered, and it is determined that embryonic nucleated red blood cells have not been recovered in a case where only one type of nucleated red blood cells have been recovered. In a case where the fetus is a male in the case where only one type of nucleated red blood cells have been recovered, or in a case where the numerical aberration of a chromosome is recognized in the case where the fetus is a female, it can be determined that embryonic nucleated red blood cells have been recovered. In a case where embryonic nucleated red blood cells have not been recovered, it is determined that a re-examination is required.

Whether or not embryonic nucleated red blood cells have been recovered may be determined by preparing the information of maternally derived cells as reference cell information in advance and performing comparison with the reference cell information.

The numerical aberration determination step S48 is a step of determining whether or not there is a numerical aberration of a specific chromosome. In the present embodiment, the presence or absence of numerical aberrations of chromosome 18 and chromosome 21 is determined by quantifying the amount of DNA belonging to the chromosome 18 and the amount of DNA belonging to the chromosome 21 and comparing the results with those in the normal case.

When determining the numerical aberration in the chromosome 21 in the numerical aberration determination step S48, a case where the quantitative value of the amount of DNA in the chromosome 21 corresponds to neither a normal range indicating normal nor an abnormal range indicating numerical aberrations is a case where a target result cannot be obtained. Accordingly, it is determined that a re-examination is required.

In the case of trisomy of the chromosome 21, the quantitative value of the amount of DNA in the chromosome 21 is 1.5 times that in the normal case. In the case of monosomy of the chromosome 21, the quantitative value of the amount of DNA in the chromosome 21 is 0.5 times that in the normal case.

A range indicating normal and a range showing abnormal are determined in consideration of an error or the like in the derivation of quantitative values. For example, the range indicating normal in the case of considering an error of 5% is equal to or greater than 0.95 times the normal value and equal to or less than 1.05 times the normal value. In the case of trisomy, the range indicating abnormal in the case of considering an error of 5% is equal to or greater than 1.43 times the normal value and equal to or less than 1.58 times the normal value.

For example, in a case where the quantitative value of the amount of DNA in the chromosome 21 is 1.2 times that in the normal case, the quantitative value of the amount of DNA in the chromosome 21 corresponds to neither the normal range indicating normal nor the abnormal range indicating numerical aberrations. Accordingly, it is determined that a re-examination is required.

Whether the examination result corresponds to the normal range and the abnormal range or does not correspond thereto may be automatically determined from the numerical values included in the examination result, or the operator may input the result of determination regarding whether the examination result corresponds to the normal range and the abnormal range or does not correspond thereto.

Whether or not embryonic nucleated red blood cells have been recovered may also be automatically determined based on determination reference information determined in advance, or the operator may input the determination result.

In a case where it is determined that a re-examination is required, re-examination necessity determination information used in determining whether or not a re-examination is required is generated. The re-examination necessity determination information also functions as information indicating that a re-examination is required. As a form of the re-examination necessity determination information, character information indicating that the possibility of success of re-examination is high or character information indicating that the possibility of success of re-examination is low can be mentioned.

In addition, the re-examination necessity determination information may always be generated regardless of whether or not a re-examination is required.

In addition, a form is possible in which information of at least one of the number of used nucleated red blood cells that is the number of used cells, a gestational age, the number of candidate nucleated red blood cells that is the number of candidate cells, the amount of blood collection, the amount of DNA, the amount of chromosomes, or a gene dosage is included as the re-examination necessity determination information. In particular, a form is preferable in which at least one of the number of used nucleated red blood cells and the gestational age is included in the re-examination necessity determination information.

The gestational age may be the number of weeks obtained by adding the date and time taken for the examination to the blood sampling date and time.

The number of used nucleated red blood cells is the number of used nucleated red blood cells used in the gene analysis step S4. The gestational age is the number of weeks of pregnancy at the time of examination. The number of candidate nucleated red blood cells is the number of nucleated red blood cells as candidates used in the gene analysis step S4 or the number of nucleated red blood cells as candidates actually used in the gene analysis step S4.

In the examination notification output step S5 shown in Fig. 1, re-examination necessity determination information generated in the sequencing step S46 and the numerical aberration determination step S48 shown in Fig. 3 is acquired together with the examination result, an examination notification reflecting the re-examination necessity determination information in the examination result is generated, and the generated examination notification is output. The details of the generation of an examination notification will be described later.

The items of the steps or the step classification method described in Figs. 1 to 3 is an example, and a method of distinguishing steps in a series of examination processes, that is, a step definition method regarding how to define each step, is arbitrary. Also for the examination process itself, it is possible to adopt other various methods. The step definition method according to the examination process is also arbitrary similarly. This is the same for a gene chromosome examination system shown below, and the definition of each unit is arbitrary and subdivision and integration of each unit can be appropriately performed.

### [Configuration of a gene chromosome examination system]

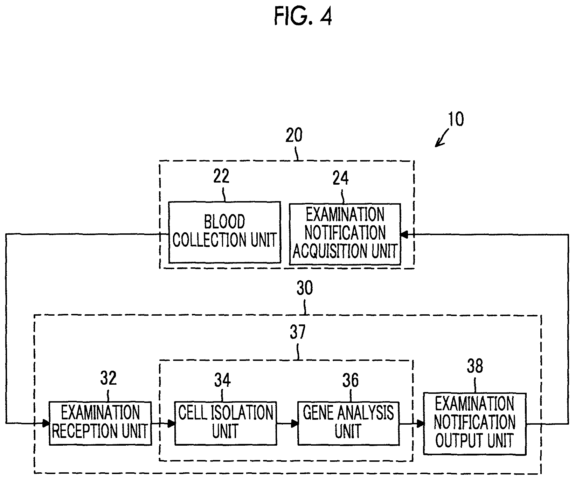

Fig. 4 is an overall configuration diagram showing the schematic configuration of a gene chromosome examination system to which the examination notification output device according to the embodiment of the present invention is applied. A gene chromosome examination system 10 shown in Fig. 4 is configured to include a hospital 20 and a laboratory 30.

The hospital 20 is configured to include a blood collection unit 22 for collecting a blood sample and an examination notification acquisition unit 24 that is an output destination of the examination notification of a gene chromosome examination. The blood sample of the mother collected by the blood collection unit 22 is sent to the laboratory 30.

Hospital information specifying the hospital 20 and maternal information specifying the mother, from whom the blood sample has been collected, are given to the blood sample of the mother sent to the laboratory 30 from the hospital 20. The hospital information and the maternal information include a form called an ID. The ID is an abbreviation of identification.

As examples of hospital information, a hospital name and blood collection date and time can be mentioned. As examples of maternal information, name, age, gestational age at the time of blood collection, and a fetal gender can be mentioned. As examples of forms of hospital information and maternal information, a form printed on the recording paper and a form of electronic data can be mentioned. A form converted into a format suitable for data communication or a form stored in a storage medium is included in the form of electronic data. A form of compression of the amount of data is included in the form converted into a format suitable for data communication.

The blood sample of the mother sent to the laboratory 30 from the blood collection unit 22 is quickly and safely transported under the temperature control and in a state of being protected against shock. For the transportation of the blood sample of the mother from the blood collection unit 22 to the laboratory 30, the transportation step S2 shown in Fig. 1 is applied.

The examination notification acquisition unit 24 shown in Fig. 4 acquires an examination notification indicating the examination result of the gene chromosome examination sent from the laboratory 30, and displays the examination notification for a person to be examined. As an example of the acquisition form of an examination notification, a form of acquiring a printed matter by printing an examination result on the recording paper or a form of acquiring an examination notification as electronic data can be mentioned. As an example of a form to visualize electronic data, printing and display using a display device can be mentioned.

The laboratory 30 is configured to include an examination reception unit 32, a cell isolation unit 34, a gene analysis unit 36, and an examination notification output unit 38. The examination reception unit 32 receives the blood sample of the mother transported from the blood collection unit 22 or the hospital 20, and acquires the hospital information and the maternal information given to the received blood sample.

The hospital information and the maternal information may be sent to the laboratory 30 separately from the blood sample using a method of data communication or the like in a state in which the hospital information and the maternal information is associated with the blood sample.

The cell isolation unit 34 performs the cell isolation step S3 shown in Fig. 1. The gene analysis unit 36 shown in Fig. 4 performs the gene analysis step S4 shown in Fig. 1. The cell isolation unit 34 and the gene analysis unit 36 shown in Fig. 4 function as an examination unit 37 that performs a gene chromosome examination.

The examination notification output unit 38 shown in Fig. 4 performs the examination notification output step S5 shown in Fig. 1. The examination notification generated by the examination notification output unit 38 shown in Fig. 4 is sent to the examination notification acquisition unit 24 belonging to the hospital 20. The examination notification output unit 38 can also belong to the examination unit 37.

In addition, the examination notification output unit 38 and the examination notification acquisition unit 24 can be integrated and placed in the hospital 20.

### [Configuration of an information processing system]

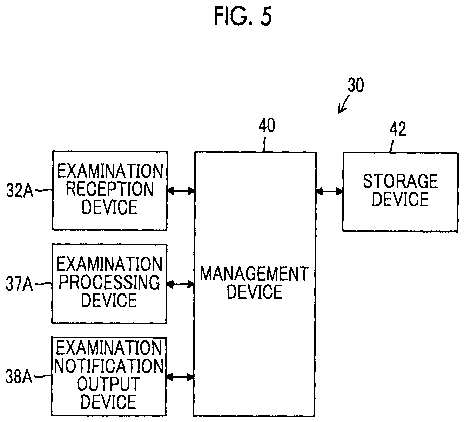

Fig. 5 is a block diagram showing the configuration of an information processing system in the laboratory 30 shown in Fig. 4. As shown in Fig. 5, the laboratory 30 includes an examination reception device 32A, an examination processing device 37A, an examination notification output device 38A, s management device 40, and a storage device 42. Overall control of each device is performed by the management device 40. The management device 40 shown in Fig. 5 can be configured as a server device disposed in the laboratory 30.

The examination reception device 32A performs a process of receiving an examination request. As a process of receiving an examination request, generation of an examination job that is a management unit of an examination in the laboratory 30, acquisition of hospital information given to a blood sample to be examined, and acquisition of maternal information given to a blood sample to be examined can be mentioned. The hospital information and the maternal information that have been acquired are associated with the examination job, and are stored in the storage device 42 through the management device 40.

In addition, it is preferable that an examination job is associated with an electronic medical record managed in the hospital 20 shown in Fig. 4. For example, an identification number or an identification mark in the electronic medical record of the mother, from whom a blood sample to be examined has been collected, may be acquired as hospital information.

As the examination reception device 32A shown in Fig. 5, a terminal device disposed in the examination reception unit 32 that functions as a processing device of the examination reception unit 32 shown in Fig. 4 is applied.

As specific examples of acquiring the hospital information and the maternal information, a form of reading stored information from an information storage medium, such as a bar code, an RFID, and an IC tag, a form of acquiring information through data communication, and a form of inputting information printed on a printed matter using an input device, such as a keyboard and a scanner, can be mentioned. Data communication may be wireless or wired.

Here, RFID is an abbreviation of radio frequency identifier, and IC is an abbreviation of integrated circuit.

The examination processing device 37A performs information processing in the examination unit 37 shown in Fig. 4. The examination processing device 37A includes a terminal device disposed in the cell isolation unit 34 shown in Fig. 4 and a terminal device disposed in the gene analysis unit 36.

The examination processing device 37A shown in Fig. 5 associates the examination result obtained by the cell isolation step S3 and the gene analysis step S4 in Fig. 1 and examination-related information, which is obtained in association with the examination, with the examination job, and stores the result in the storage device 42 through the management device 40.

As an example of the examination-related information, the re-examination necessity information and the re-examination necessity determination information that have been described previously can be mentioned. As other examples of the examination-related information, information regarding the state of a blood sample, such as the contamination of a blood sample, and information regarding the type of numerical aberration of a specific chromosome, such as mosaic chromosomal abnormalities, partial trisomy, or chimera, can be mentioned.

The examination notification output device 38A performs the examination notification output step S5 shown in Fig. 1. The examination notification output device 38A acquires the examination result and the re-examination necessity determination information or the examination-related information stored in the storage device 42, generates an examination notification based on the examination result, and outputs the generated examination notification.

In the storage device 42, various kinds of information for each examination job are stored. The examination reception device 32A, the examination processing device 37A, or the examination notification output device 38A accesses the storage device 42 to store and read the various kinds of information.

Access of the examination reception device 32A, the examination processing device 37A, or the examination notification output device 38A to the storage device 42 is managed by the management device 40. The management device 40 may set the access permission for the examination reception device 32A, the examination processing device 37A, or the examination notification output device 38A, so that it is possible to access only a region determined for each device or only information determined for each device.

The configuration of the information processing system of the laboratory 30 shown in Fig. 5 is an example, and the functions of the respective devices can be appropriately integrated and the functions of each device can be appropriately divided. For example, the examination notification output device 38A may be made to have some of the functions of the examination processing device 37A.

In addition, any of the examination reception device 32A, the examination processing device 37A, and the examination notification output device 38A may also be combined with the management device 40.

The gene chromosome examination system 10 shown in Fig. 4 may be constructed as a client server type computer system, or may be configured as a system of a client that does not use a server.

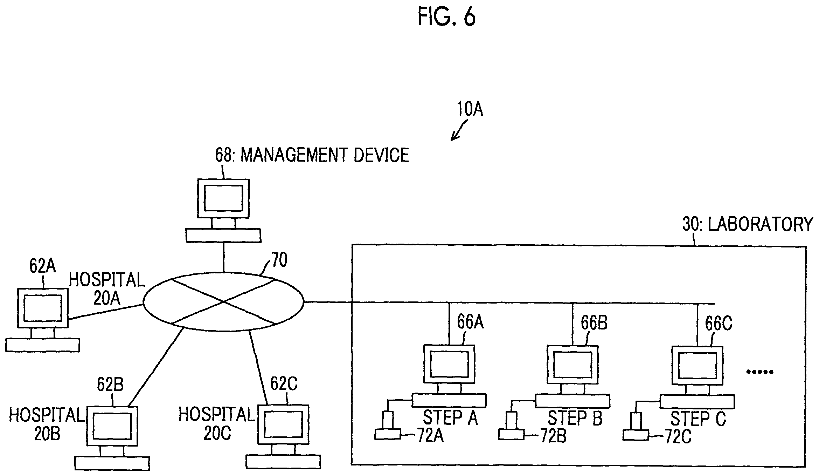

Fig. 6 is a block diagram of a gene chromosome examination system 10A to which a client server type computer system is applied.

The gene chromosome examination system 10A shown in Fig. 6 is configured to include a management device 68, a hospital terminal device 62A installed in a hospital 20A, a hospital terminal device 62B installed in a hospital 20B, a hospital terminal device 62C installed in a hospital 20C, and a laboratory terminal device 66 installed in the laboratory 30.

The management device 68 shown in Fig. 6 has a function of the management device 40 shown in Fig. 5. The hospital terminal devices 62A, 62B, and 62C shown in Fig. 6 can be made to function as the terminal device of the blood collection unit 22 and the terminal device of the examination notification acquisition unit 24 shown in Fig. 4.

A laboratory terminal device installed in each unit of the laboratory 30 is included in the laboratory terminal device 66. In Fig. 6, a laboratory terminal device 66A applied to a process A, a laboratory terminal device 66B applied to a process B, and a laboratory terminal device 66C applied to a process C are shown as the laboratory terminal device 66.

For example, the laboratory terminal devices 66A, 66B, and 66C shown in Fig. 6 can be made to function as the examination reception device 32A, the examination processing device 37A, and the examination notification output device 38A shown in Fig. 5, respectively.

The management device 68 that functions as a server device is communicably connected to the hospital terminal devices 62A, 62B, and 62C, which function as client devices, and the laboratory terminal devices 66A, 66B, and 66C through electrical communication lines.

The hospital terminal devices 62A, 62B, and 62C shown in Fig. 6 may function as the examination notification output device 38A shown in Fig. 5. In a case where the hospital terminal devices 62A, 62B, and 62C shown in Fig. 6 function as the examination notification output device 38A shown in Fig. 5, the hospital terminal device that functions as the examination notification output device 38A receives only an examination result from the laboratory terminal device that functions as the examination processing device 37A. The hospital terminal device that functions as the examination notification output device 38A may receive an examination result from the laboratory terminal device, which functions as the examination processing device 37A, through the examination reception device 32A.

An electrical communication line 70 may be a local area network, or may be a wide area network, or may be a combination thereof. The local area network may be expressed as a LAN using the first letters of local area network of English notation. The wide area network may be expressed as a WAN using the first letters of wide area network of English notation.

The electrical communication line 70 is not limited to a cable communication line. A part or all of the electrical communication line 70 can be formed as a wireless communication line. The term "connection" in this specification indicates a state of devices between which transmission and reception of a signal are possible. As a form of connection, any of wired connection and wireless connection can be applied.

Although the hospital terminal device 62A installed in the hospital A, the hospital terminal device 62B installed in the hospital B, and the hospital terminal device 62C installed in the hospital C are shown in Fig. 6, there is no particular limitation on the number of hospital terminals when implementing the invention, and any number (1 or more) of hospital terminals can be set. In addition, it is also possible to adopt a configuration in which a plurality of hospital terminals are installed in the same hospital.

In addition, although the case where the number of laboratories 30 is one is exemplified in Fig. 6, there is no particular limitation on the number of laboratories 30, and any number (1 or more) of laboratories 30 can be set. In this case, the management device 68 manages each of the plurality of laboratories 30 separately.

Bar code readers 72A, 72B, and 72C are connected to the laboratory terminal devices 66A, 66B, and 66C, respectively. Although not shown in Fig. 6, a bar code including identification information for identifying a sample is attached to a container or slide glass to receive a blood sample. The bar code is read by the bar code readers 72A, 72B, and 72C when starting the work or processing of each step or when ending the work or processing of each step or at the timing of each of the start and the end.

In the present embodiment, a bar code reader is exemplified as means for reading hospital information and maternal information from the information storage medium in which the hospital information and the maternal information in a blood sample to be examined are stored. However, means for reading hospital information and maternal information corresponding to the configuration of an information storage medium is provided.

The gene chromosome examination system 10A shown in Fig. 6 is an example in a case where the client server type computer system is applied, and addition of a configuration, change of a configuration, and deletion of a configuration can be appropriately performed.

### [Explanation of an examination notification output device]

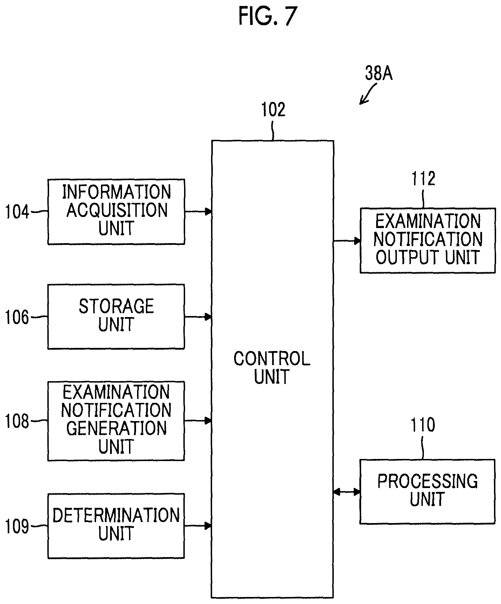

The examination notification output device 38A shown in Fig. 5 will be described in detail. Fig. 7 is a block diagram showing the configuration of the examination notification output device 38A shown in Fig. 5.

The examination notification output device 38A shown in Fig. 7 is configured to include a control unit 102, an information acquisition unit 104 that functions as information acquisition means, a storage unit 106, an examination notification generation unit 108 that functions as examination notification generation means, a determination unit 109, a processing unit 110, and an examination notification output unit 112 that functions as an examination notification output means.

The control unit 102 performs overall control of each unit of the examination notification output device 38A. In addition, the control unit 102 functions as a memory controller that controls the writing of data into the storage unit 106 and the reading of data from the storage unit 106.

The information acquisition unit 104 acquires at least an examination result. The information acquisition unit 104 may acquire information required for the processing of the examination notification output device 38A including information indicating that a re-examination is required, for example, re-examination necessity determination information, or a second information acquisition unit that acquires information other than an examination result may be provided.

As an example of information acquisition by the information acquisition unit 104, information required for processing may be read from the storage device 42 by accessing the storage device 42 through the management device 40 shown in Fig. 5.

Information, such as the re-examination necessity determination information and the examination result acquired by the information acquisition unit 104, is stored in the storage unit 106.

The examination notification generation unit 108 generates an examination notification based on the information that has been acquired by the information acquisition unit 104 and is stored in the storage unit 106. In a case where it is determined that a re-examination is required, the examination notification generation unit 108 generates an examination result including the re-examination necessity determination information. The examination notification generated by the examination notification generation unit 108 is sent to the examination notification output unit 112 through the control unit 102.

The determination unit 109 performs processing for determining whether or not a re-examination is required from the examination result acquired by the information acquisition unit 104. In a case where information indicating whether or not a re-examination is required is acquired by the information acquisition unit 104, the determination unit 109 may be removed.

The processing unit 110 performs information processing when generating the examination notification from the information acquired by the information acquisition unit 104. The processing unit 110 and the determination unit 109 can also be integrated.

The examination notification output unit 112 outputs the examination notification generated by the examination notification generation unit 108. As examples of the output form of the examination notification, electronic data, a printed material, and the like can be mentioned. The examination notification output from the examination notification output unit 112 is sent to the examination notification acquisition unit 24 belonging to the hospital 20 shown in Fig. 4.

The examination notification output device 38A shown in Fig. 7 is an example, and addition of a configuration, change of a configuration, and deletion of a configuration can be appropriately performed.

### [Explanation of examination notification]

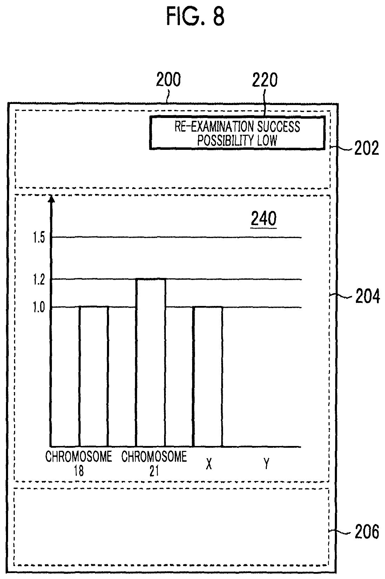

Next, specific examples of the examination notification will be described with reference to Figs. 8 to 12. The examination notifications shown in Figs. 8 to 10 are sent in a case where the examination result corresponds to neither the normal range nor the abnormal range, which is an example in a case where it is determined that a re-examination is required.

Fig. 8 is an explanatory diagram showing an example of the examination notification sent in a case where the examination result corresponds to neither the normal range nor the abnormal range. An examination notification 200 is configured to include a first region 202, a second region 204, and a third region 206. In the first region 202, re-examination success possibility information 220 is displayed.

As the re-examination success possibility information 220 shown in Fig. 8, character information expressing the possibility of success of re-examination in two stages is applied. For the re-examination success possibility information 220, any expression form can be applied if the re-examination success possibility information 220 is information indicating the possibility of success of re-examination. For example, it is possible to apply an expression form using multi-stage numerical values or an expression form using symbols or the like.

Quantitative value information 240 indicating the quantitative value of the amount of DNA in a specific chromosome is displayed in the second region 204. In Fig. 8, the quantitative value of the amount of DNA of the chromosome 18 as a specific chromosome, the quantitative value of the amount of DNA of the chromosome 21, and the quantitative value of the amount of DNA of sex chromosome are displayed in a graph form.

The horizontal series of the quantitative value information 240 indicates a chromosome number and the type of sex chromosome. The vertical series of the quantitative value information 240 is a quantitative value of the amount of DNA. As the quantitative value of the amount of DNA, a relative value when the quantitative value of the amount of DNA in a normal case where there is no numerical aberration of a chromosome is assumed to be 1.0 is applied.

In the examination notification 200 shown in Fig. 8, the quantitative value information 240 indicating the quantitative value of the amount of DNA in a specific chromosome is applied as information indicating the presence or absence of numerical aberration of a specific chromosome that is included in the examination result.

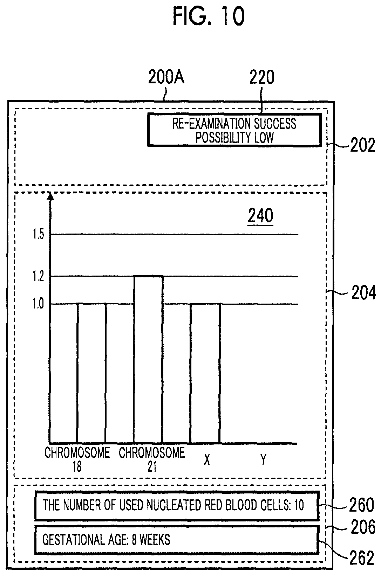

In the third region 206, examination execution conditions information indicating the examination execution conditions in the gene analysis step S4 shown in Fig. 1 is displayed. In the examination notification 200 shown in Fig. 8, nothing is displayed in the third region 206. The examination execution conditions information is shown by reference numerals 260 and 262 in Fig. 10.

The re-examination success possibility information 220 displayed in the first region 202, the quantitative value information 240 displayed in the second region 204, and the examination execution conditions information displayed in the third region 206 function as re-examination necessity determination information used in determining whether or not a re-examination is required.

In the examination notification 200 shown in Fig. 8, the quantitative value of the amount of DNA in the chromosome 21 is 1.2 times that in the normal case. If numerical aberration occurs in the chromosome 21, the quantitative value of the amount of DNA in the chromosome 21 becomes 0.5 times or 1.5 times that in the normal case.

However, the quantitative value of the amount of DNA of the chromosome 21 shown in Fig. 8 corresponds to neither 0.5 times nor 1.5 times that in the normal case. That is, since the examination result corresponds to neither the normal range nor the abnormal range, it is difficult to determine whether or not numerical aberration of the chromosome 21 has occurred from the content of the quantitative value information 240.

Therefore, by outputting the examination notification 200 including the re-examination success possibility information 220 as re-examination necessity determination information, whether or not the possibility of success of re-examination is high can be grasped at a glance from the re-examination success possibility information 220.

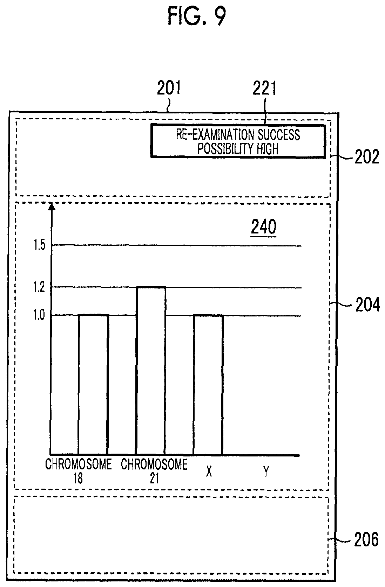

Fig. 9 is an explanatory diagram showing another example of the examination notification sent in a case where the examination result corresponds to neither the normal range nor the abnormal range. The examination notification 201 shown in Fig. 9 includes re-examination success possibility information 221 indicating that the possibility of success of re-examination is high, instead of the re-examination success possibility information 220 shown in Fig. 8 indicating that the possibility of success of re-examination is low.

Thus, in a case where the examination result of the gene chromosome examination corresponds to neither the normal range nor the abnormal range, an examination notification including the re-examination necessity determination information is output. Accordingly, the re-examination necessity determination information can be used as an indicator when determining whether or not to perform re-examination.

At least the re-examination success possibility information 220 may be given to the examination notification 200 shown in Fig. 8 by omitting the quantitative value information 240. In addition, at least the re-examination success possibility information 221 may be given to the examination notification 201 shown in Fig. 9 by omitting the quantitative value information 240.

Fig. 10 is an explanatory diagram showing another example of the examination notification in a case where the examination result corresponds to neither the normal range nor the abnormal range. In Fig. 10, the same or similar components as in Fig. 8 are denoted by the same reference numerals, and the explanation thereof will be appropriately omitted.

In an examination notification 200A shown in Fig. 10, the examination execution conditions information 260 and 262 that functions as re-examination necessity determination information is added to the examination notification 200 shown in Fig. 8.

In the examination notification 200A shown in Fig. 10, the number of used nucleated red blood cells that is the number of used cells is displayed as the examination execution conditions information 260, and the gestational age is displayed as the examination execution conditions information 262. The value of the number of used nucleated red blood cells and the value of the gestational age shown in Fig. 10 are arbitrary values. This is the same in the following diagrams.

In the examination notification 200A shown in Fig. 10, the examination execution conditions information 262 itself can also be displayed in the first region 202 as the re-examination success possibility information 220. In a case where the examination execution conditions information 262 itself is displayed in the first region 202 as the re-examination success possibility information 220, character information expressing the possibility of success of re-examination in two stages may be omitted. A doctor can determine the possibility of success of the re-examination just by checking the examination execution conditions information 262.

The number of used nucleated red blood cells is the number of nucleated red blood cells that have been used to quantify the amount of DNA in the gene chromosome examination by which the examination result has been acquired. The gestational age is the number of weeks of pregnancy at the time of gene chromosome examination.

As other examination execution conditions information that functions as re-examination necessity determination information, the amount of blood collection and the number of candidate nucleated red blood cells can be mentioned. The number of candidate nucleated red blood cells is the number of nucleated red blood cells as candidates for nucleated red blood cells used in the gene chromosome examination.

If the number of nucleated red blood cells relative to the amount of blood collection is small compared with a standard range, it can be determined that the possibility of success of re-examination is low even if the re-examination is performed without increasing the amount of blood collection. Accordingly, it is possible to perform determination, such as increasing the amount of blood collection in the re-examination from the previous examination.

As the causes of "examination result of a gene chromosome examination corresponds to neither a normal range nor an abnormal range", the influence of measurement error, mosaic chromosomal abnormalities, partial trisomy, contamination after blood collection, contamination before blood collection, chimera, and the like can be mentioned.

The mosaic chromosomal abnormalities are a state in which normal cells and abnormal cells are mixed. The partial trisomy is trisomy in which chromosomes partially overlap each other. The contamination is a state in which other blood components are mixed to the collected blood sample.

The chimera is a state in which cells with different pieces of genetic information are mixed. The chimera occurs in a case where two fertilized eggs are fused in the womb or in a case where hematopoietic tube cells have moved in twins, for example. The former occurs very rarely. The latter is referred to as a blood chimera, and occurs in about 8% of the dizygotic twins pair.

By displaying the number of used nucleated red blood cells as re-examination necessity determination information, the number of used nucleated red blood cells can be used as an indicator for determining whether or not a re-examination is required in a case where the influence of measurement error and mosaic chromosomal abnormalities are suspected. For example, in a case where the number of used nucleated red blood cells shown as the examination execution conditions information 260 is insufficient compared with the standard number of the number of used nucleated red blood cells determined in advance, it can be determined that the possibility of success of re-examination is high on the condition that the number of nucleated red blood cells is increased in the re-examination.

In order to determine whether or not it is possible to increase the number of used nucleated red blood cells, the gestational age, the amount of blood collection, and the number of candidate nucleated red blood cells can be used as indicators. For example, since the number of nucleated red blood cells tends to increase with the passage of gestation from the fifth week to the tenth week of pregnancy, the gestational age can be used as an indicator for determining whether or not it is possible to increase the number of used nucleated red blood cells.

In addition, also in a case where embryonic nucleated red blood cells have not been recovered, the number of used nucleated red blood cells serves as an indicator when determining the possibility of success of re-examination. That is, if it is possible to adjust a period up to the re-examination, it can be determined that the possibility of success of the re-examination is high.

If it is possible to increase the amount of blood collection or the number of candidate nucleated red blood cells, a possibility that it is possible to increase the number of used nucleated red blood cells becomes high. Therefore, the amount of blood collection or the number of candidate nucleated red blood cells can be used as an indicator for determining whether or not it is possible to increase the number of used nucleated red blood cells.

In a case where the contamination after blood collection is suspected, it can be determined that the possibility of success of re-examination is high on the condition that the re-examination is performed from the blood collection step. As an indicator for determining whether or not it is possible to perform the re-examination from the blood collection step, the gestational age can be used. In addition, the gestational age can be used as an indicator when determining the necessity of re-examination in consideration of time limitation of abortion.

In Fig. 10, as a display example of the third region 206, a form is exemplified in which the examination execution conditions information 260 and 262 is displayed. However, at least one of the pieces of examination execution conditions information 260 and 262 may be included. In addition, as the examination execution conditions information, at least one of the amount of blood collection and the number of candidate nucleated red blood cells may be added.

The arrangement and sizes of the first region 202, the second region 204, and the third region 206 shown in Figs. 8 to 10 can be appropriately changed.

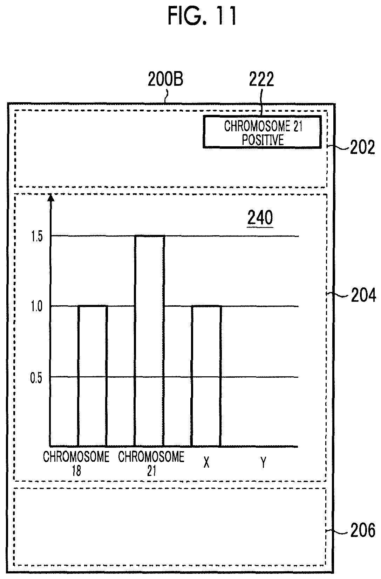

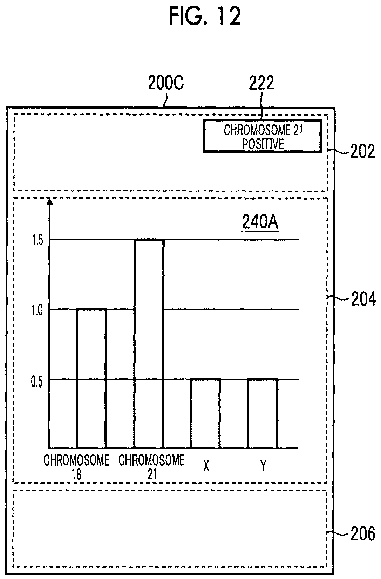

Figs. 11 and 12 are explanatory diagrams showing examples of the notification of an examination result in a case where a re-examination is not required, that is, in a case where an examination result, from which the presence or absence of numerical aberration of a specific chromosome can be determined, is obtained and the examination is successful. An examination notification 200B shown in Fig. 11 is a case where the fetus is a female, and an examination notification 200C shown in Fig. 12 is a case where the fetus is a male.

In a case where the fetus shown in Fig. 11 is a female, the amount of DNA in the sex chromosome displayed as the quantitative value information 240 has only a quantitative value of the amount of DNA in the X chromosome. In a case where the fetus shown in Fig. 12 is a male, the amount of DNA in the sex chromosome displayed as the quantitative value information 240A has a quantitative value of the amount of DNA in the X chromosome and a quantitative value of the amount of DNA in the Y chromosome.

In addition, in a case where the fetus shown in Fig. 12 is a male, the amount of DNA in the X chromosome and the amount of DNA in the Y chromosome are half of the amount of DNA in the X chromosome in a case where the fetus shown in Fig. 11 is a female.

In the examination notification 200B shown in Fig. 11 and the examination notification 200C shown in Fig. 12, determination result information 222 indicating the presence or absence of numerical aberration of a specific chromosome is given to the first region 202. In both of the examination notification 200B shown in Fig. 11 and the examination notification 200C shown in Fig. 12, character information indicating that the quantitative value of the amount of DNA in the chromosome 21 is a value indicating positive is displayed.

Even in a case where a re-examination is not required, the examination execution conditions information 260 shown in Fig. 10 may be displayed in the first region 202 or the third region 206 as the re-examination success possibility information 220.

This is because, even in a case where information indicating that a re-examination is not required is displayed in the first region 202, the doctor may determine that re-examination is to be performed for relief depending on the numerical value of the quantitative value information 240.

### [Explanation of the control procedure of an examination notification output method]

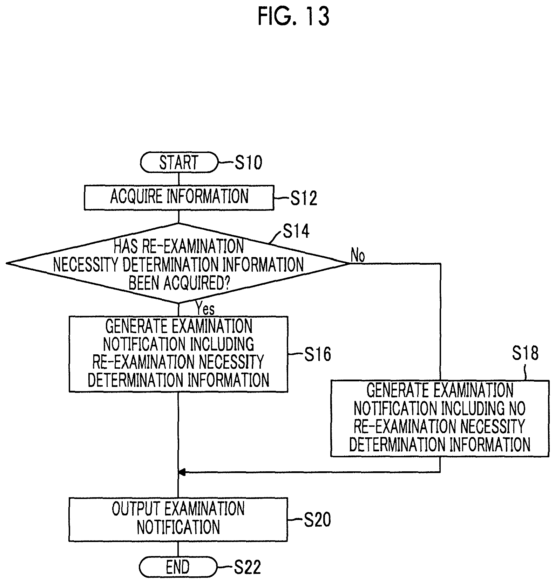

Fig. 13 is a flowchart showing the control procedure of an examination notification output method. When the control of the examination notification output method is started in start step S10, information acquisition step S12 is performed. In the information acquisition step S12, information including at least an examination result is acquired.

In a case where it is determined that a re-examination is required, for example, in a case where an examination result indicating that the examination result of a gene chromosome examination corresponds to neither the normal range nor the abnormal range is obtained, information including re-examination necessity determination information, for example, the re-examination success possibility information 220 shown in Fig. 8, is acquired together with the examination result. When the information is acquired in the information acquisition step S12, the process proceeds to examination notification selection step S14.

In the examination notification selection step S 14, it is checked whether or not re-examination necessity determination information is included in the information acquired in the information acquisition step S12, and the type of examination notification is selected according to whether or not the re-examination necessity determination information is included.

The examination result may be acquired in the information acquisition step S12, and it may be determined whether or not the examination result corresponds to neither the normal range nor the abnormal range and the type of examination notification may be selected in the examination notification selection step S14.

In a case where re-examination necessity determination information is included in various kinds of information acquired in the information acquisition step S12 and Yes is determined in the examination notification selection step S14, an examination notification including the re-examination necessity determination information is selected, and the process proceeds to a first examination notification generation step S16.

On the other hand, in a case where re-examination necessity determination information is not included in various kinds of information acquired in the information acquisition step S12 and No is determined in the examination notification selection step S14, an examination notification including no re-examination necessity determination information is selected, and the process proceeds to a second examination notification generation step S18.

In the case of automatically determining whether or not a re-examination is required based on the examination result, it is determined whether or not a re-examination is required in the examination notification selection step S 14. When it is determined that a re-examination is required, the process proceeds to the first examination notification generation step S16. When it is determined that a re-examination is not required, the process proceeds to the second examination notification generation step S18.

In the first examination notification generation step S16, an examination notification including re-examination necessity determination information, such as the examination notification 200 shown in Fig. 8, is generated. In the second examination notification generation step S18, an examination notification including no re-examination necessity determination information, such as the examination notification 200B shown in Fig. 11, is generated.

When the examination notification is generated in the first examination notification generation step S16 or in the second examination notification generation step S18, the process proceeds to an examination notification output step S20. In the examination notification output step S20, the examination notification is output. After the examination notification is output, the process proceeds to end step S22 to end the examination notification output method.

### [Explanation of an examination notification output program]

It is also possible to configure an examination notification output program corresponding to the examination notification output device and the examination notification output method that have been described previously.

For example, it is possible to configure an examination notification output program causing a computer to function as information acquisition means for acquiring information including the examination result of a gene chromosome examination, examination notification generation means for generating an examination notification by adding re-examination necessity determination information, which is used in determining whether or not a re-examination is required, to the examination result, and examination notification output means for outputting the generated examination notification.

That is, it is possible to configure an examination notification output program causing a computer to function as means corresponding to each unit of the examination notification output device 38A.

According to the examination notification output device, the examination notification output method, and the examination notification output program configured as described above, an examination notification is generated by adding the re-examination necessity determination information, which is used in determining whether or not a re-examination is required, to the examination result, and the examination notification is output. Therefore, in the determination regarding whether or not to perform re-examination, it is possible to use the re-examination necessity determination information as an indicator.

In a case where region-examination is required, which is a case where an examination result indicating that the examination result of a gene chromosome examination corresponds to neither the normal range nor the abnormal range is obtained, an examination notification is generated by adding re-examination necessity determination information to the examination result, and the examination notification is output. Therefore, in particular, in the determination regarding whether or not to perform re-examination in a case where a re-examination is required, it is possible to use the re-examination necessity determination information as an indicator.

### [Explanation of other examples of the configuration of examination notification]

Next, other examples of the configuration of the examination notification will be described. In the following explanation, the same or similar components as in the examination notification described previously are denoted by the same reference numerals, and the explanation thereof will be appropriately omitted.

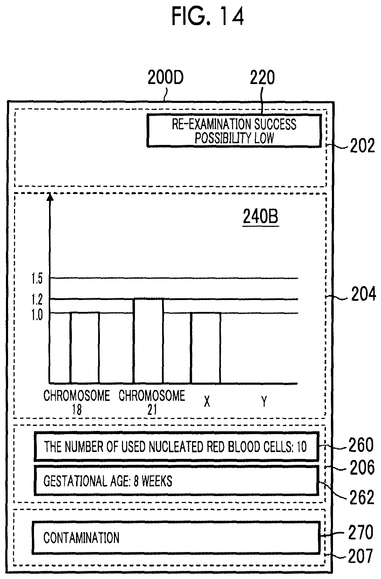

Fig. 14 is an explanatory diagram of an examination notification including reason information indicating the determination reason for re-examination success determination. In an examination notification 200D shown in Fig. 14, reason information 270 is given to the fourth region 207. As examples of the determination reason, contamination shown in Fig. 14 or partial trisomym, chimera, and the like that are not shown in Fig. 14, can be mentioned. As other examples of the determination reason, non-recovery of embryonic nucleated red blood cells can be mentioned. In a case where information including determination reason information is acquired, it is possible to add the reason information 270.

By adding the reason information 270 to the examination notification 200D, it is possible to use the reason information 270 as an indicator for determining whether or not a re-examination is required.

The quantitative value information 240B of the examination notification 200D shown in Fig. 14 is reduced in size compared with the quantitative value information 240 of the examination notification 200 shown in Fig. 8. In the examination notification 200D, the fourth region 207 is added on the side of the third region 206 opposite to the second region 204. In the examination notification 200D including the reason information 270, the re-examination success possibility information 220 may be omitted. Thus, the layout of the examination notification may be appropriately changed according to items displayed on the examination notification.

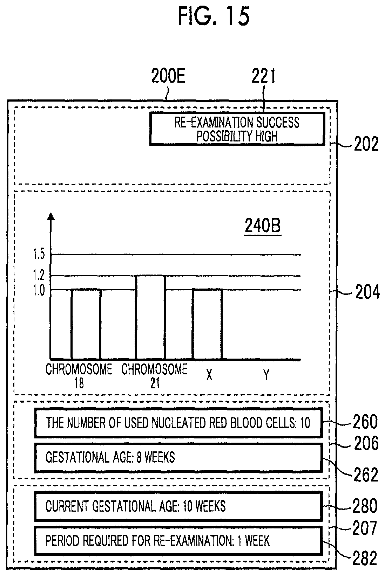

Fig. 15 is an explanatory diagram of an examination notification including supplementary information that supplements the re-examination necessity determination information. In an examination notification 200E shown in Fig. 15, information of the current gestational age is given to the fourth region 207 as first supplementary information 280, and information of a period required for a re-examination is given as second supplementary information 282.

The gestational age displayed as examination execution conditions information 262 shown in Fig. 15 is the gestational age at the time of blood collection that is when blood has been collected from the mother, that is, the gestational age when the examination has been performed.

The current gestational age is the gestational age of the mother at the time of examination notification output when the examination notification is displayed by the examination notification acquisition unit 24 shown in Fig. 4.

The gestational age of the mother at the time of examination notification output is calculated by acquiring the current date and time when the examination result is displayed, calculating the elapsed time from the date and time when the examination has been performed, and converting the calculated elapsed time into the number of weeks.

In a case where it is not possible to calculate the time when the examination notification is displayed by the examination notification acquisition unit 24, the gestational age when the examination notification is acquired by the examination notification acquisition unit 24, when the examination notification is generated by the examination notification output unit 38, or when the examination notification is output may be given.

By displaying the current gestational age on the examination notification, the doctor can determine whether or not to perform re-examination, for example, even in a case where the possibility of success of re-examination is high in terms of the number of nucleated red blood cells.

In a case where the current gestational age is displayed on the examination notification, by displaying the information of a period required for re-examination in the case of performing re-examination on the examination notification, the doctor can perform determination taking into consideration what kind of determination can be made at the timing of acquiring the examination notification of re-examination.

In Fig. 15, a form is exemplified in which two kinds of supplementary information of the first supplementary information 280 and the second supplementary information 282 are given. However, the number of supplementary information, the content of supplementary information, and the like can be appropriately changed, added, or deleted.

In the examination notification 200E shown in Fig. 15, the re-examination success possibility information 221 indicating that the possibility of success of re-examination is high is given as the re-examination necessity determination information.

By adding the supplementary information to the examination notification, it is possible to use the supplementary information as an indicator for determining whether or not a re-examination is required. In addition, it is possible to use the supplementary information as an indicator for determining the conditions of a re-examination, such as performing re-examination early.

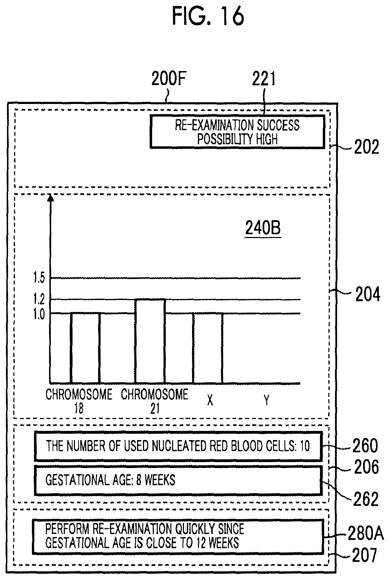

Fig. 16 is an explanatory diagram of another example of supplementary information. In an examination notification 200F shown in Fig. 16, since the gestational age at the time of examination is close to 12 weeks, character information indicating the quick execution of re-examination is given as supplementary information 280A. The character information functions as information that is an indicator for setting the conditions of a re-examination in a case where the re-examination is performed.

In the examination notification 200F shown in Fig. 16, the re-examination success possibility information 221 indicating the possibility of success of re-examination is high is given as the re-examination necessity determination information.

Although not shown, in the examination notification 200F shown in Fig. 16, the re-examination success possibility information 220 indicating the possibility of success of re-examination is low may be given as the re-examination necessity determination information, and character information indicating that re-examination is not performed may be given as the supplementary information 280A.

By appropriately selecting the content of supplementary information included in the examination notification, the supplementary information can be used as the conditions of a re-examination based on the examination result, for example, an indicator for determining the priority of re-examination. Specifically, it is possible to urge to perform blood collection for re-examination on the same day.

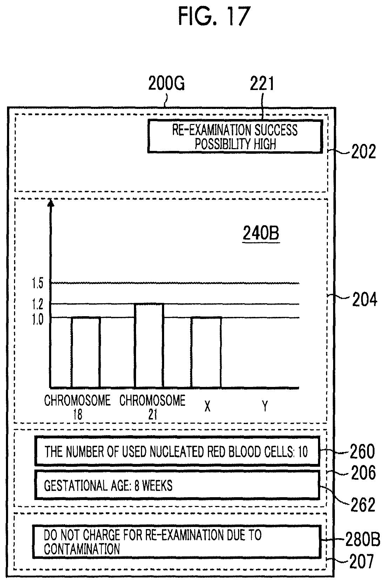

Fig. 17 is an explanatory diagram of another example of supplementary information. An examination notification 200G shown in Fig. 17 includes information regarding a charge in the case of performing re-examination. That is, supplementary information 280B indicates no charge when performing re-examination.

In the examination notification 200G shown in Fig. 17, the re-examination success possibility information 221 indicating the possibility of success of re-examination is high is given as the re-examination necessity determination information.

For example, in a case where contamination occurs after blood collection in the laboratory 30 not at the time of blood collection in the hospital 20 shown in Fig. 5, a response, such as making no additional charge, is possible at the time of re-examination. Also in a case where the third or subsequent examinations occur, a response, such as making no additional charge for a predetermined number of re-examinations, is possible.

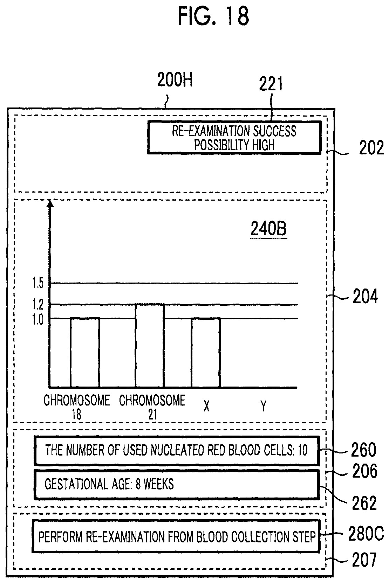

Fig. 18 is an explanatory diagram of another example of supplementary information. In an examination notification 200H shown in Fig. 18, character information indicating the step of a gene chromosome examination from which a re-examination is started is given as supplementary information 280C. As a step from which a re-examination is started, the blood collection step S1 or the gene analysis step S4 shown in Fig. 1 can be mentioned.

In the examination notification 200H shown in Fig. 18, the re-examination success possibility information 221 indicating the possibility of success of re-examination is high is given as the re-examination necessity determination information.

As a case of performing re-examination from the blood collection step S1, contamination after blood collection can be mentioned. Also in a case where the lack of the number of candidate nucleated red blood cells is concerned, it is possible to perform re-examination from the blood collection step S1.

As a case of performing re-examination from the gene analysis step S4 that is an intermediate step, mosaic chromosomal abnormalities can be mentioned. In the case of mosaic chromosomal abnormalities, re-examination may be performed from the blood collection step S1 in order to increase the number of candidate nucleated red blood cells in the re-examination.

Also in a case where the number of candidate nucleated red blood cells is a sufficient number and the lack of the number of used nucleated red blood cells is concerned, it is possible to perform the re-examination from the gene analysis step S4 by adding nucleated red blood cells to be examined using candidate nucleated red blood cells that have not been examined.

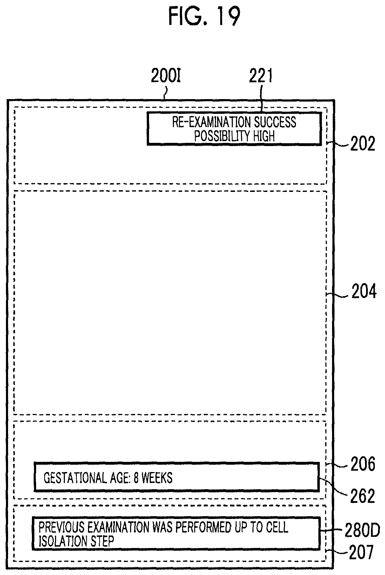

Fig. 19 is an explanatory diagram of another example of supplementary information. In an examination notification 200I shown in Fig. 19, character information indicating the step of a gene chromosome examination, in which it has been determined that an examination result indicating that the examination result corresponds to neither the normal range nor the abnormal range has been obtained, is given as supplementary information 280D.

In the examination notification 200I shown in Fig. 19, the re-examination success possibility information 221 indicating the possibility of success of re-examination is high is given as the re-examination necessity determination information.

For example, in a case where it has been determined that a re-examination is required in the cell isolation step S3 shown in Fig. 1, the examination is stopped at the point in time when it has been determined that a re-examination is required, and the examination notification 200I shown in Fig. 19 is generated and output.

As other examples, a case where a necessary and sufficient number of nucleated red blood cells could not be secured in the cell isolation step S3 or the examination step after the cell isolation step S3 shown in Fig. 1 can be mentioned. In a case where the examination is stopped at the point in time when it has been determined that a re-examination is required, information obtained until the stopped step is displayed on the examination notification.

Since the examination is stopped at the point in time when it has been determined that a re-examination is required and the examination notification 200I is generated and output, determination regarding whether or not to perform re-examination can be performed earlier. Accordingly, in a case where a re-examination is performed, it is possible to perform the re-examination earlier.

The pieces of supplementary information shown in Figs. 15 to 19 can be appropriately combined. When applying a plurality of pieces of supplementary information, the size of each character used for the supplementary information may be changed, or the size of the fourth region 207 may be changed.

Color display can be applied to the examination notifications shown in Figs. 8 to 12 and 14 to 19. By displaying information, which needs to be distinguished visually, with a different color, the information that needs to be distinguished visually can be emphasized more than other pieces of information. In addition, one examination notification may be configured using a plurality of pages.

### [Explanation of handling of examination notification]

The examination notification acquisition unit 24 shown in Fig. 4 acquires the examination notification sent from the examination notification output unit 38. In a case where the examination notification 200B shown in Fig. 11 and the examination notification 200C shown in Fig. 12 are acquired, the determination result information 222 indicating the presence or absence of numerical aberration of a specific chromosome is given.

On the other hand, in a case where an examination notification including re-examination necessity determination information, such as the examination notification 200 shown in Fig. 8, is acquired, it is possible to ask a person to be examined about whether or not to have a re-examination.

When determining whether or not to perform re-examination or when selecting an option in the re-examination, the examination execution conditions information 260 and 262 of the examination notification 200A shown in Fig. 10 can be used. For example, in a case where the number of used nucleated red blood cells or the number of candidate nucleated red blood cells is less than the standard value or in a case where embryonic nucleated red blood cells have not been recovered, it is possible to propose an option, such as increasing the amount of blood collection in the re-examination compared with that in the previous examination.

In addition, in a case where the number of used nucleated red blood cells or the number of candidate nucleated red blood cells is less than the standard value or in a case where embryonic nucleated red blood cells have not been recovered, it is possible to propose an option of setting a period up to the re-examination in consideration of the gestational age in order to increase the number of nucleated red blood cells in the mother.

In addition, in a case where the gestational age is close to 12 weeks, for example, in a case where the gestational age is 11 weeks, it is possible to propose an option of performing no re-examination or to propose an option of performing re-examination preferentially.

The supplementary information 280B shown in Fig. 17 is an indicator for determining a charge in the re-examination. In addition, the supplementary information 280C shown in Fig. 18 and the supplementary information 280D shown in Fig. 19 are indicators for determining the step from which a re-examination is started.

### [Explanation of re-examination]

In a case where a re-examination is performed, a blood sample arrives at the laboratory 30 through the blood collection step S1 and the transportation step S2 shown in Fig. 1. Information that indicates a re-examination and that is associated with the previous examination is added to the hospital information or the maternal information given to a blood sample.

Since the hospital information and the maternal information may be anonymized, it is difficult to check to which examination job the re-examination corresponds in the laboratory 30. By adding the information from which it is possible to understand that the re-examination is associated with the previous examination, it is possible to perform the examination preferentially by reason of re-examination. Therefore, it is possible to use information acquired in the previous examination.

In the present embodiment, the gene chromosome examination using a blood sample has been described with the non-invasive prenatal diagnosis as an example. However, the output device and the like according to the present embodiment can also be applied to a genetic examination using a sample other than blood samples, such as a mucosal cell.

In the examination notification output device, the examination notification output method, the examination notification output program, and the gene chromosome examination system described above, appropriate changes, addition, and deletion are possible without departing from the spirit and scope of the present invention. In addition, the embodiments described above can also be appropriately combined.

### Explanation of References

10, 10A: gene chromosome examination system
32: examination reception unit
32A: examination reception device
38: examination notification output unit
38A: examination notification output device
40: management device
42: storage device
104: information acquisition unit
108: examination notification generation unit
109: determination unit
112: examination notification output unit
200, 200A, 200B, 200C, 200D, 200E, 200F, 200G, 200H, 200I, 201: examination notification
220, 221: re-examination success possibility information
240: quantitative value information
260, 262: examination execution conditions information
270: determination reason
280: first supplementary information
282: second supplementary information
280A, 280B, 280C, 280D: supplementary information

## Claims

1. An examination notification output device that outputs an examination notification of a gene chromosome examination for examining abnormalities of chromosomes included in embryonic cells, comprising:
an information acquisition unit that acquires information including an examination result of a gene chromosome examination;
an examination notification generation unit that generates an examination notification by adding re-examination necessity determination information, which is used in determining whether or not a re-examination is required, to the examination result; and
an examination notification output unit that outputs the generated examination notification.

2. The examination notification output device according to claim 1,
wherein the information acquisition unit acquires information including at least one of a number of used cells indicating a number of cells used in the gene chromosome examination, a gestational age of a mother at the time of the gene chromosome examination, a number of candidate cells indicating a number of cells as candidates that are used in the gene chromosome examination, an amount of blood collection, an amount of DNA, an amount of chromosomes, or a gene dosage, and
the examination notification generation unit adds at least one of the pieces of information acquired, as the re-examination necessity determination information, to the examination notification.

3. The examination notification output device according to claim 1 or 2,
wherein the examination notification generation unit adds re-examination success possibility information indicating a possibility that re-examination will be successful, as the re-examination necessity determination information, to the examination notification.

4. The examination notification output device according to claim 3,
wherein the information acquisition unit acquires reason information indicating a determination reason for the re-examination success possibility information, and
the examination notification generation unit adds the acquired reason information to the examination notification.

5. The examination notification output device according to any one of claims 1 to 4,
wherein the information acquisition unit acquires information of a gestational age of the mother at the time of examination notification output, and
the examination notification generation unit adds the acquired gestational age of the mother at the time of examination notification output to the examination notification.

6. The examination notification output device according to any one of claims 1 to 5,
wherein the information acquisition unit acquires information of a period required for a re-examination in a case where the re-examination is performed, and
the examination notification generation unit adds the acquired period, which is required for the re-examination in a case where the re-examination is performed, to the examination notification.

7. The examination notification output device according to any one of claims 1 to 6,
wherein the information acquisition unit acquires information that is an indicator for setting conditions of a re-examination in a case where the re-examination is performed, and
the examination notification generation unit adds the acquired information, which is an indicator for setting the conditions of the re-examination, to the examination notification.

8. The examination notification output device according to any one of claims 1 to 7,
wherein the information acquisition unit acquires information regarding a charge in a case where a re-examination is performed, and
the examination notification generation unit adds the acquired information regarding a charge to the examination notification.

9. The examination notification output device according to any one of claims 1 to 8,
wherein the information acquisition unit acquires information of a step of the gene chromosome examination, in which it has been determined that an examination result indicating that a re-examination is required has been obtained, and
the examination notification generation unit adds the acquired information of the step of the gene chromosome examination, in which it has been determined that an examination result indicating that a re-examination is required has been obtained, to the examination notification.

10. The examination notification output device according to any one of claims 1 to 9,
wherein the information acquisition unit acquires information of a step of the gene chromosome examination, from which a re-examination is started in a case where the re-examination is performed, and
the examination notification generation unit adds the acquired information of the step of the gene chromosome examination, from which a re-examination is started, to the examination notification.

11. The examination notification output device according to any one of claims 1 to 10,
wherein, in a case where it is determined that a re-examination is required based on the acquired examination result, an examination notification including the re-examination necessity determination information is generated.

12. The examination notification output device according to claim 11,
wherein, in a case where the acquired examination result corresponds to neither a normal range nor an abnormal range or in a case where the acquired examination result does not correspond to an examination result in embryonic cells, the examination notification generation unit generates an examination notification including re-examination necessity determination information, which is used in determining whether or not a re-examination is required, in a case where it is determined that a re-examination is required based on the acquired examination result.

13. The examination notification output device according to claim 11 or 12,
wherein the information acquisition unit acquires information indicating a case where it has been determined that a re-examination is required.

14. The examination notification output device according to any one of claims 11 to 13, further comprising:
a determination unit that determines whether or not a re-examination is required based on the acquired information.

15. An examination notification output method for outputting an examination notification of a gene chromosome examination for examining abnormalities of chromosomes included in embryonic cells, comprising:
an information acquisition step of acquiring information including an examination result of a gene chromosome examination;
an examination notification generation step of generating an examination notification by adding re-examination necessity determination information, which is used in determining whether or not a re-examination is required, to the examination result; and
an examination notification output step of outputting the generated examination notification.

16. An examination notification output program causing a computer to execute the examination notification output method according to claim 15.

17. An examination notification output program for outputting an examination notification of a gene chromosome examination for examining abnormalities of chromosomes included in embryonic cells, the program causing a computer to function as:
information acquisition means for acquiring information including an examination result of a gene chromosome examination;
examination notification generation means for generating an examination notification by adding re-examination necessity determination information, which is used in determining whether or not a re-examination is required, to the examination result; and
examination notification output means for outputting the generated examination notification.

18. A gene chromosome examination system for examining abnormalities of chromosomes included in embryonic cells, comprising:
an examination unit that performs a gene chromosome examination of acquiring a blood sample collected from a mother, recovering candidate cells, which are candidates for embryonic cells, from the acquired blood sample, and performing gene analysis using the recovered candidate cells; and
an examination notification output device that outputs an examination notification of the gene chromosome examination performed by the examination unit,
wherein the examination notification output device comprises:
an information acquisition unit that acquires information including an examination result of a gene chromosome examination;
an examination notification generation unit that generates an examination notification by adding re-examination necessity determination information, which is used in determining whether or not a re-examination is required, to the examination result; and
an examination notification output unit that outputs the generated examination notification.

19. The gene chromosome examination system according to claim 18,
wherein the examination notification output device includes the examination notification output device according to any one of claims 2 to 14.
